Europäisches Patentamt

(19) European Patent Office

Office européen des brevets

(11) Publication number : **0 167 506**
**B1**

(12) **EUROPEAN PATENT SPECIFICATION**

(45) Date of publication of patent specification :
19.10.88

(21) Application number : 85850186.9

(22) Date of filing : 28.05.85

(51) Int. Cl.⁴ : **A 61 B 19/08**

(54) **Surgical drape and a method of manufacturing it.**

(30) Priority : 29.05.84 SE 8402904

(43) Date of publication of application :
08.01.86 Bulletin 86/02

(45) Publication of the grant of the patent :
19.10.88 Bulletin 88/42

(84) Designated contracting states :
BE DE FR GB NL

(56) References cited :
FR-A- 2 171 386
US-A- 2 593 121
US-A- 2 715 902
US-A- 3 251 360
US-A- 3 750 663
US-A- 3 769 971
US-A- 3 856 005
US-A- 3 862 632
US-A- 3 911 499
US-A- 4 196 723
US-A- 4 327 448

(73) Proprietor : **Mölnlycke AB**
**S-405 03 Göteborg (SE)**

(72) Inventor : **Hanssen, Carl-Otto**
**Västervägen 4**
**S-43041 Kullavik (SE)**
Inventor : **Starzmann, Martin**
**Pontus Wiknersgatan 6**
**S-41132 Göteborg (SE)**

(74) Representative : **Alfredson, Stig et al**
**H. ALBIHNS PATENTBYRA AB Box 3137**
**S-103 62 Stockholm (SE)**

## Description

The present invention relates to a surgical drape intended to cover a patient during surgical incisions in the urethra/vagina and/or in the abdomen. The invention also relates to a method of producing such a surgical drape.

When performing surgical procedures in the urethra/vagina, it has been found difficult so far to provide a sufficient degree of sterility in the area of surgical incisions on the patient as well as on the operating table because of the position of the patient during such surgical procedures with the legs widely spread apart and elevated while being supported in specific stirrups. A particular slitted drape has been used which is more closely described below. The use of this previously known slitted drape however involves numerous shortcomings. In fact, it is not possible to provide coverage around the operative area solely by using this type of drape. The open slit of the drape therefore has to be covered by a separate surgical sheet. Furthermore, the covering of the patient's legs will not extend all the way down to the floor ; the bottom edge thereof will instead terminate at a considerable height above the floor, causing in this manner the risk of contamination. In addition, the geometry of the prior art surgical drape is not properly adapted to the specific resting position of the patient, which makes it practically impossible when applying the drape to prevent the sheet from being creased within the area around the cutout or operating opening, which in turn creates problems for the surgeon with respect to accessibility at the area of incision.

US-A-3 862 632 describes a surgical drape intended to cover a patient during incisions in the urethra/vagina and/or the abdomen, comprising a portion serving to cover the upper part of the patient's body, and two leggings having widened portions serving to cover each leg from the foot up to the knee of a patient resting with the legs widely spread apart, raised and bent at the knees, as well as tapering portions intended to cover each leg from the knee up to the patient's crotch. Such a drape however is fabricated from three pieces of material.

The above-mentioned disadvantages with prior art surgical drapes for use during incisions in the urethra/vagina and/or abdomen have however been entirely overcome with the present invention, primarily in that the inventive surgical drape is designed in one single piece with a rectangular portion serving to cover the patient above the waist, and a pair of leggings each of which having widened portions intended to cover one leg from foot to knee of a patient lying with the legs widely spread apart, raised and bent at the knees, as well as a tapering portion intended to cover each leg from the knee and up to the crotch of the patient, this pair of leggings being integral from the crotch of the leggings and down to the bottom edge of the drape, at least one operating opening being made in this drape for providing access to incisions in the urethra/vagina and/or abdomen. Furthermore, a surgical drape in accordance with the invention is easily manufactured from one single piece by means of the process defined in Claim 5.

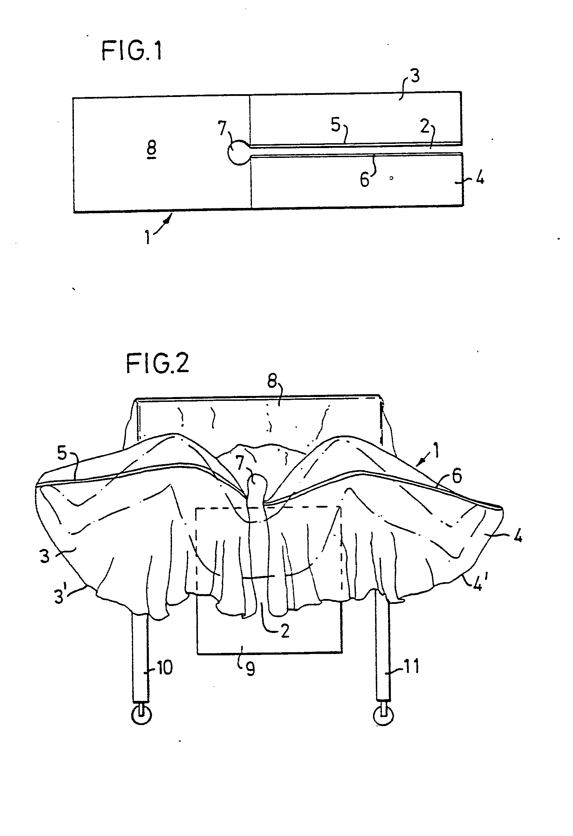

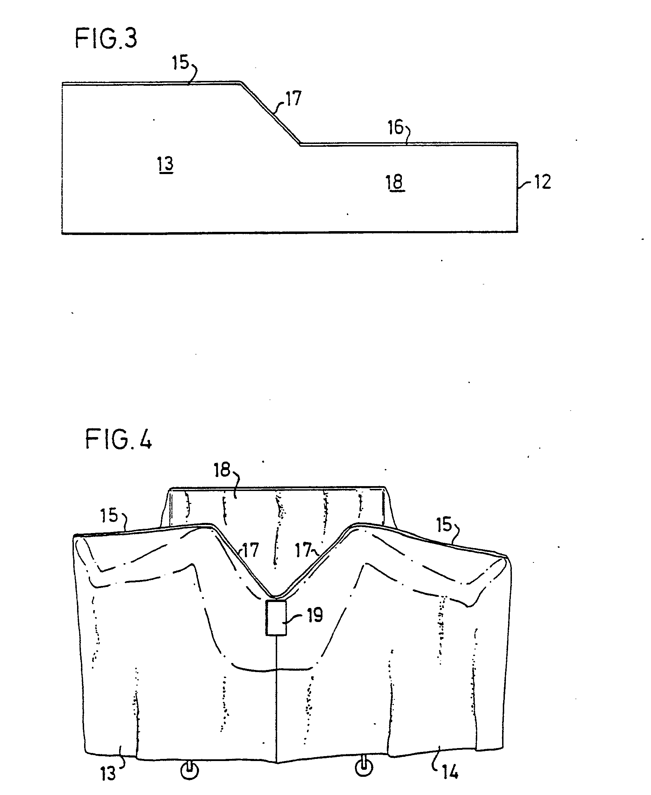

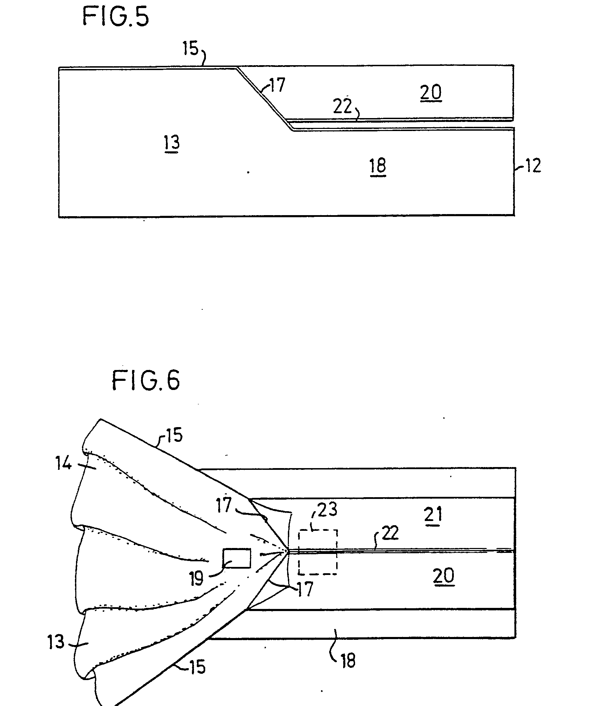

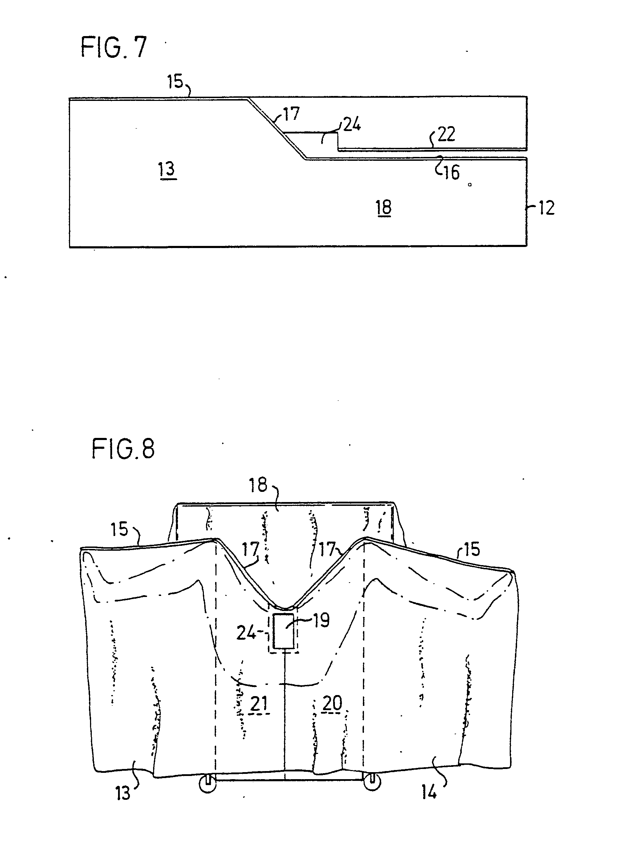

The invention will be described in more detail while referring to the embodiments illustrated in the accompanying drawings. Fig. 1 of the drawings is a plan view of a prior art surgical drape, Fig. 2 is a perspective view of the surgical drape of Fig. 1 during use, Fig. 3 is a view of an embodiment of a surgical drape made in accordance with the invention and shown in a planar, folded condition, Fig. 4 is a perspective view corresponding to Fig. 2 of the inventive surgical drape of Fig. 3 while being used, Fig. 5 is a view corresponding to that of Fig. 3 of a somewhat modified embodiment·of a surgical drape made according to the invention, Fig. 6 is a view from above of the modified drape shown in Fig. 5 in its folded-out condition, Fig. 7 is a view corresponding to Fig. 5 showing the inventive drape provided with an operating opening, whereas Fig. 8 finally is a perspective view of the drape shown in Fig. 7 when used during an urethra operation.

The prior art surgical drape shown in Fig. 1 is formed as one single elongate piece which is divided along a substantial portion of its length by a longitudinal slit 2. The portions 3, 4 disposed at either side of the slit are folded and stiched together along their edges facing the slit. The seams are denoted 5 and 6 in the drawing. The folded portions 3, 4 are intended to cover the patient's legs during use of the drape. At the inner end of the slit there is made a cutout 7 constituting an operating opening during surgical procedures. The wider portion 8 of the surgical drape serves partly to cover the body of the patient above the legs, and partly to be applied over an anesthetic frame.

Fig. 2, which is a perspective view as seen in the direction towards the area of incision, illustrates the positioning of the prior art drape shown in Fig. 1. The legs of the patient are indicated by dash-dotted lines in this figure from which it is also evident that the leggings 3, 4 are not adapted to the position of the patient's legs during the operation. It appears from Fig. 1 that the folded portions 3, 4 are quite straight, whereas the legs of the patient as viewed in Fig. 2 are distinguishly bent. As a consequence, the leggings 3, 4 of the drape are not well-adapted to the position of the patient's legs but are instead creased thereby forming heavy folds which disturbingly affect the surgical procedure. The open gap formed by the slit 2 must of course be covered as well, which is done by using a separate sheet 9. Furthermore, the leggings 3, 4 of the prior art drape have narrow dimensions resulting in that the lower edges 3', 4' thereof will be hanging high up above the floor, causing in this way a considerable risk for the operating staff to bump directly into the legs 10, 11 of the operating table or any other unprotected object below said edges 3', 4'. If the staff then touch their pants or protective coats in order to wipe their hands for example, there is a great danger of the operation wound being contaminated with bacteria coming from the uncovered objects below the edges 3', 4' of the sheet.

Fig. 3 illustrates a suitable embodiment of a surgical drape in a folded condition, made in accordance with the invention. This drape is manufactured from one single elongate piece which is folded around its transverse center line, the continuous end portion formed by folding being folded inwardly in the direction towards the opposite end 12 which is left open due to folding, obtaining thereby two leggings 13, 14 of which one 13 is indicated in Fig. 3. After folding, the blank has been sheared or cut longitudinally in the direction from the open end 12 parallel with the longitudinal direction of the blank and up to the leggings 13, 14 formed by folding, and thereafter obliquely outwards towards one edge 15 of the blank and the respective legging. The straight and oblique cutting edges are denoted 16 and 17 in Fig. 3. The folded and cut or sheared blank has then been united alongside the cutting edges 16 and along the longitudinal edges 15 and the oblique edges 17 of each legging. In this manner there is partly obtained a rectangular sheet portion 18 which is intended to cover the portion of the patient's body above the legs as well as being hung over the anesthetic frame conventionally used in surgery, and partly the leggings 13, 14 which are almost tent-shaped and intended to cover the legs of the patient as well as the region around the area of incision. Prior to or after folding, an operating opening has been made in the blank which in the completed drape shown herein is located in the crotch portion between the leggings 13, 14. This operating opening is illustrated in Fig. 4 and is denoted there by 19.

Fig. 4 illustrates the application of the drape described here while referring to Fig. 3. The legs of the patient are indicated by dash-dotted lines in the figure, and this figure as a whole constitutes a perspective view seen in the direction towards the area of incision. As is clearly evident from Fig. 4, the geometry of the drape leggings 13, 14 is adapted to the position of the patient's legs during surgical incisions in urethra or in the proximity thereof. The oblique edge 17 of either legging 13, 14 rests against the upper side of the patient's upper leg portion, whereas the joining edge 15 of the legging extends from the knee down to the foot of the patient. Thanks to this arrangement, the portion of the surgical drape disposed around the operating opening 19, i. e. the sides of the drape leggings 13, 14 facing the operating personnel, will be completely smooth, as seen in Fig. 4. Due to the leggings 13, 14 being united at the crotch, there is no need of any additional cover sheets, in contradiction to the case with prior art surgical drapes. As seen in Fig. 4, the leggings 13, 14 are preferably so widely dimensioned that they reach all the way down to the floor during use, and thereby the risk of contaminating the operation wound is considerably decreased in comparison with the risk involved with the use of prior art drapes according to Figs. 1 and 2.

Fig. 5 illustrates a somewhat modified embodiment of the drape shown in Fig. 3. The details of Fig. 5 which correspond to similar components in Fig. 3 have been given the same reference numerals as those of the latter figure. As in the embodiment according to Fig. 3, the manufacture of the surgical drape illustrated in Fig. 5 is based on the use of one single elongate, rectangular piece. The only essential difference as compared to Fig. 3 is that the drape portions 20, 21 disposed outside the cutting edge 16 extending in the longitudinal direction of the drape are united by welding to the oblique edge 17 of each legging 13, 14. For enabling separation of the leggings, the remaining triangular flap of the originally first fold which was subsequently folded inwards, is cut perpendicularly inwardly from the upper longitudinal side down to its apex which is located at the point where the edges 16 and 17 meet. The additional drape portions 20, 21 situated outside the leggings are furthermore welded together along the longitudinally cut edge 22. The flap formed by said drape portions 20, 21 can then be turned between two positions; one where it is turned over and in between the leggings 13, 14 for covering the operating opening 19, and one where it is positioned across the rectangular drape portion 18 and covers an operating opening 23 therein, as is shown in Fig. 6. The surgical drape shown in Figs. 5 and 6 thus constitutes a combination drape which can be utilized for incisions in the vagina/urethra as well as in the abdomen.

Fig. 7 illustrates a drape corresponding to the embodiment shown in Fig. 5 but having an operating opening 24 made in the flap member which is formed of the portions 21, 22 disposed outside the leggings 13, 14. This drape is intended to be turned inside out prior to use. After being turned in this manner, the flap formed by the portions 20, 21 is laid inside the leggings 13, 14 as shown in Fig. 8, the operating opening 24 of the flap thereby ending up right ahead of the operating opening 19 in the crotch of the leggings. The zone around the operating opening 24 on the side of the flap facing the patient during use is preferably provided with a binding agent allowing the flap, which can also be introduced a distance underneath the patient's buttocks, to be attached at a predetermined part of the patient's body.

Thus, the flap made out of the united portions 20 and 21 is utilized as a sterile screening of the patient, and when using the surgical drape described with reference to Figs. 7 and 8, the physician who is sitting between the drape leggings 13, 14 during surgery will be able, without further regard to a sterile set-up, to place the legs under the leggings of the surgical drape.

A surgical drape performed in accordance with the present invention is however not restricted to the exemplary embodiments set forth above, since a plurality of modifications are conceivable within the scope of the following claims.

## Claims

1. A surgical drape intended to cover a patient during incisions in the urethra/vagina and/or the abdomen, comprising a portion (18), serving to cover the upper part of the patient's body, and two leggings (13, 14) having widened portions serving to cover each leg from the foot up to the knee of a patient resting with the legs widely spread apart, raised and bent at the knees, as well as tapering portions intended to cover each leg from the knee up to the patient's crotch, characterized in that the drape is manufactured as one single piece with said portion (18) being rectangular and the leggings (13, 14) being integral with each other from the crotch of the leggings and down to the lower edge of the drape wherein there is made at least one operating opening (19, 23, 24) for the performance of surgical incisions in the urethra/vagina and/or the abdomen.

2. A drape according to Claim 1, characterized in that it is manufactured from one single elongate piece which is folded around its transverse center line, the continuous end portion thus obtained being folded inwards for creating two leggings (13, 14) dimensioned for enabling each legging to accommodate one of the patient's legs ; that the drape has a straight longitudinal edge constituting the lower edge when using the drape ; that the folded blank has at its open end (12) a narrower portion (18) with a center line (16) running parallel to the lower edge and extending up to the leggings ; that the upper edge of the folded blank thereafter extends obliquely outwards a distance (17) substantially corresponding to the length of the upper leg portion of a patient and that the rest of the upper edge (15) of each legging is substantially parallel to the lower edge of the drape ; that the folded blank is joined along the center line (16) of the narrow portion and that the leggings are individually joined alongside the oblique edges (17) and further along the upper edge sections (15) up to their closed ends.

3. A drape according to Claim 1, characterized in that it is manufactured from one single elongate rectangular piece which is folded around its transverse center line, its continuous end portion obtained in this manner being folded inwards to form a pair of leggings (13, 14) dimensioned for enabling one of the patient's legs to be accommodated in each legging ; that the drape has a straight longitudinal edge constituting the lower edge when using the drape ; that the folded blank is sheared or cut up from its open end along a cutting line (22) running in parallel to the longitudinal edge up to the leggings (13, 14) ; that the drape is joined together at either side of said cutting line to form the rectangular portion (18) as well as a flap (20, 21) ; that the folded blank is joined together from the termination of the cutting line (22) along a line running obliquely outwards a distance (17) substantially corresponding to the length of the patient's upper leg portion and that the rest of the upper edge (15) of each legging, which is substantially parallel to the lower edge of the drape, is joined along the upper edge sections (15) up to the closed ends of the leggings.

4. A drape according to Claim 3, characterized in that operating openings (19, 23) are made between the leggings (13, 14) as well as in the rectangular portion (18) in the proximity of the leggings ; and that the flap (20, 21) is intended to be applied over one or the other of the two operating openings depending on whether a surgical incision is to be performed in the urethra/vagina or in the abdomen.

5. A method of producing a surgical drape according to Claim 1 to be used during surgical incisions in the urethra/vagina and/or the abdomen, characterized in that the drape is made out of one single elongate blank which is folded around its transverse center line, the continuous folding end obtained being folded inwards to form two leggings (13, 14) dimensioned to enable one of the patient's legs to be accommodated in each legging, the blank thereafter being sheared or cut longitudinally in the direction from its end (12) which is left open due to the folding, parallel to the longitudinal direction of the blank and at least substantially up to the leggings (13, 14) ; that the folded blank is joined along the sheared or cut edge (16) ; and that the leggings (13, 14) are individually joined to said cut edge (16) obliquely outwards from one edge of the blank or legging and further along straight edges (15) up to the closed ends of the leggings, at least one through-going operating opening (19) being made in the blank.

6. A method according to Claim 5, characterized in that the drape is produced of a rectangular blank ; that portions (20, 21) above the cutting line (16) are joined (22) along said line to form a continuous flap (20, 21) intended to cover either an operating opening (19) disposed at the crotch of the leggings, or an operating opening (3) made in the rectangular portion (18).

7. A method according to Claim 5, characterized in that the blank, after having been longitudinally sheared or cut in the direction from the end of the blank (12) which is left open due to the folding, parallel to the longitudinal direction of the blank and at least substantially up to the leggings (13, 14), is thereafter sheared or cut obliquely outwards towards one edge (15) of the blank and each legging, the folded blank then being joined along the sheared or cut edge (16) ; and that the leggings (13, 14) are individually joined along oblique edges (17) and further along straight edges (15) up to the closed ends of the leggings, there being made in the blank a through-going operating opening (19) which in the completed surgical drape is located between the leggings, primarily at the crotch of the leggings.

## Patentansprüche

1. Chirurgisches Abdecktuch zum Abdecken eines Patienten während des Eingriffs in die Harnröhre/Vagina und/oder in den Bauch, umfassend einen Abschnitt (18) zum Abdecken des oberen Teils des Körpers des Patienten und zwei Beinteile (13, 14) mit erweiterten Abschnitten zum Abdecken jedes Beines vom Fuss bis zum Knie eines Patienten, der mit weit gespreizten, angehobenen und abgebogenen Knien ruht, wobei sich verjüngende Abschnitte jedes Bein vom Knie bis hinaus zum Schritt des Patienten bedecken, dadurch gekennzeichnet, dass das Abdecktuch als ein einziges Stück mit dem genannten Abschnitt (18), der rechteckig ist, hergestellt ist, und wobei die Beinteile (13, 14) vom Schritt der Beinteile und hinunter zur Unterkante des Abdecktuchs, in dem zumindest eine Operationsöffnung (19, 23, 24) zum Durchführen des chirurgischen Eingriffs in die Harnröhre/Vagina und/oder den Bauch ausgebildet ist, miteinander integral sind, ausgebildet ist.

2. Abdecktuch nach Anspruch 1, dadurch gekennzeichnet, dass es aus einem einzelnen länglichen Stück hergestellt ist, welches um seine Quermittellinie gefaltet ist, dass der so erhaltene kontinuierliche Endabschnitt zur Schaffung der zwei Beinteile (13, 14) einwärts gefaltet ist, wobei die beiden Beinteile so dimensioniert sind, dass jedes Beinteil ein Bein des Patienten aufnimmt, dass das Abdecktuch eine gerade Längskante hat, die bei Verwendung des Abdecktuches die Unterkante bildet, dass das offene Ende (12) des gefalteten Zuschnitts einen schmäleren Abschnitt (18) mit einer parallel zur Unterkante und hinauf zu den Beinteilen verlaufenden Mittellinie (16) aufweist, dass die Oberkante des gefalteten Zuschnitts danach über eine Wegstrecke (17) schräg nach aussen verläuft, die im wesentlichen der Länge des oberen Beinabschnittes eines Patienten entspricht, wobei der Rest der Oberkante (15) jedes Beinteils im wesentlichen parallel zur Unterkante des Abdecktuches verläuft, dass der gefaltete Zuschnitt entlang der Mittellinie (16) des schmalen Abschnittes verbunden ist, und dass die Beinteile für sich entlang der schräg verlaufenden Kanten (17) und weiter entlang der oberen Kantensektionen (15) bis zu ihren geschlossenen Enden zusammengefügt sind.

3. Abdecktuch nach Anspruch 1, dadurch gekennzeichnet, dass es aus einem einzelnen, länglichen, rechtwinkligen Stück hergestellt ist, welches um seine Quermittellinie gefaltet ist, wobei der auf·diese Weise erzielte kontinuierliche Endabschnitt zur Bildung eines Paares von Beinteilen (13, 14) einwärts gefaltet ist, welche Beinteile so dimensioniert sind, dass ein Bein des Patienten von jedem Beinteil aufgenommen wird, dass das Abdecktuch eine gerade Längskante aufweist, die im benutzten Zustand die Unterkante bildet, dass der gefaltete Zuschnitt von seinem offenen Ende entlang einer Schnittlinie (22), die parallel zur Längskante hinauf bis zu den Beinteilen (13, 14) verläuft, aufgeschnitten ist, dass das Abdecktuch an jeder Seite der Schnittlinie zusammengefügt ist, um den rechtwinkligen Abschnitt (18) sowie einen Lappen (20, 21) zu bilden, dass der gefaltete Zuschnitt vom Ende der Schnittlinie (22) über eine Wegstrecke (17), die im wesentlichen der Länge des oberen Beinabschnittes des Patienten entspricht entlang einer schräg nach aussen verlaufenden Linie zusammengefügt ist, und dass der Rest der oberen Kante (15) jedes Beinabschnittes, welcher im wesentlichen parallel zur Unterkante des Abdecktuches verläuft, entlang der oberen Kantensektionen·(15) bis zu den geschlossenen Enden der Beinteile zusammengefügt ist.

4. Abdecktuch nach Anspruch 3, dadurch gekennzeichnet, dass die Operationsöffnungen (19, 23) zwischen den Beinteilen (13, 14) ebenso wie in dem rechtwinkligen Abschnitt (18) in der Nähe der Beinteile ausgebildet sind, und dass der Lappen (20, 21) dazu bestimmt ist, über die eine oder die andere der beiden Operationsöffnungen gelegt zu werden, und zwar in Abhängigkeit davon, ob ein chirurgischer Eingriff in der Harnröhre/Vagina oder im Bauch vorgenommen wird.

5. Verfahren zum Herstellen eines chirurgischen Abdecktuches nach Anspruch 1 für die Verwendung während chirurgischer Eingriffe in die Harnröhre/Vagina und/oder den Bauch, dadurch gekennzeichnet, dass das Abdecktuch aus einem einzelnen länglichen Zuschnitt gemacht ist, welches um seine Quermittellinie gefaltet ist, wobei das so erhaltene, kontinuierlich gefaltete Ende zur Ausbildung von zwei Beinteilen (13, 14), die so dimensioniert sind, dass sie jeweils ein Bein des Patienten aufnehmen, einwärts gefaltet wird, dass der Zuschnitt danach in Richtung von seinem Ende (12), welches aufgrund des Faltens offen gelassen wurde, parallel zur Längsrichtung des Zuschnittes und zumindest im wesentlichen bis zu den Beinteilen (13, 14) aufgeschnitten wird, dass der gefaltete Zuschnitt entlang der Schnittkante (16) zusammengefügt wird, und dass die Beinteile (13, 14) mit der Schnittkante (16) schräg nach aussen von einer Kante des Zuschnittes oder des Beinteils und weiter entlang der geraden Kanten (15) bis zu den geschlossenen Enden der Beinteile individuell verbunden werden, wobei zumindest eine Durchgangs-Operationsöffnung (19) im Zuschnitt ausgeführt wird.

6. Verfahren nach Anspruch 5, dadurch gekennzeichnet, dass das Abdecktuch aus einem rechtwinkligen Zuschnitt erzeugt wird, dass Abschnitte (20, 21) oberhalb der Schnittlinie (16) entlang dieser Linie verbunden werden, um einen kontinuierlichen Lappen (20, 21) zum Abdecken entweder der im Schritt der Beinteile befindlichen Operationsöffnung (19) oder der im rechtwinkligen Abschnitt (18) ausgeführten Operationsöffnung (3) zu bilden.

7. Verfahren nach Anspruch 5, dadurch gekennzeichnet, dass der Zuschnitt nach dem Längsdurchschneiden in Richtung von dem Ende des Zuschnittes (12), welches beim Falten offen gelassen wurde, parallel zur Längsrichtung des Zuschnittes und zumindest im wesentlichen bis zu den Beinteilen (13, 14) danach schräg nach aussen in Richtung auf eine Kante (15) des Zuschnittes und jedes Beinteils aufgeschnitten wird, dass der gefaltete Zuschnitt dann entlang der Schnittkante (16) zusammengefügt wird, und dass die Beinteile (13, 14) entlang der schrägen Kanten (17) und weiter entlang der geraden Kanten (15) bis zu den geschlossenen Enden der Beinteile individuell angeschlossen sind, wobei im Zuschnitt eine Durchgangs-Operationsöffnung (19) gemacht ist, welche sich im fertiggestellten chirurgischen Abdecktuch zwischen den Beinteilen, in erster Linie im Schritt der Beinteile, befindet.

## Revendications

1. Tissu chirurgical destiné à recouvrir un patient pendant incision de l'urètre/vagin et/ou de l'abdomen, comprenant une partie (18), servant à couvrir la partie supérieure du corps du patient, et deux jambières (13, 14) présentant des parties élargies servant à couvrir chaque jambe depuis le pied jusqu'au genou d'un patient reposant les jambes largement écartées, remontées et fléchies aux genoux, ainsi que des parties évasées destinées à couvrir chaque jambe depuis le genou jusqu'à l'entrejambe du patient, caractérisé en ce que le tissu est produit en une seule pièce, ladite partie (18) étant rectangulaire, et les jambières (13, 14) étant d'une seule pièce depuis l'entrejambe des jambières jusqu'au bord inférieur du tissu dans lequel est ménagée au moins une ouverture opératoire (19, 23, 24) permettant de pratiquer des incisions chirurgicales dans l'urètre/vagin et/ou l'abdomen.

2. Tissu selon la revendication 1, caractérisé en ce qu'il est produit en partant d'une seule pièce oblongue qui est pliée autour de sa médiane transversale, le tronçon extrême continu ainsi obtenu étant replié vers l'intérieur pour former deux jambières (13, 14) dimensionnées en sorte que chaque jambière puisse recevoir une des jambes du patient ; en ce que le tissu présente un bord longitudinal rectiligne constituant le bord inférieur lors de l'utilisation du champ ; en ce que l'ébauche pliée présente à son extrémité ouverte (12) une partie plus étroite (18) à médiane (16) s'étendant parallèlement au bord inférieur et allant vers le haut jusqu'aux jambières ; en ce que le bord supérieur de l'ébauche pliée s'étend ensuite obliquement vers l'extérieur sur une distance (17) correspondant sensiblement à la longueur de la partie supérieure de la jambe d'un patient et en ce que le reste du bord supérieur (15) de chaque jambière est sensiblement parallèle au bord inférieur du champ ; en ce que l'ébauche pliée est réunie suivant la médiane (16) de la partie étroite et en ce que les jambières sont jointes individuellement le long des côtés obliques et en outre le long des sections marginales supérieures (15) jusqu'à leurs extrémités fermées.

3. Tissu selon la revendication 1, caractérisé en ce qu'il est produit en partant d'une seule pièce rectangulaire oblongue qui est pliée autour de sa médiane transversale, son tronçon d'extrémité continu obtenu de cette manière étant replié vers l'intérieur pour former deux jambières (13, 14) de dimensions voulues pour permettre de loger une jambe du patient dans chaque jambière ; en ce que le champ présente un bord longitudinal rectiligne constituant le bord inférieur lors de l'utilisation du champ ; en ce que l'ébauche pliée est cisaillée ou sectionnée à partir de son extrémité ouverte suivant une ligne de coupe (22) s'étendant parallèlement au bord longitudinal jusqu'aux jambières (13, 14) ; en ce que le champ est réuni de part et d'autre de cette ligne de coupe pour constituer la partie rectangulaire (18) ainsi qu'un rabat (20, 21) ; en ce que l'ébauche pliée est rattachée depuis la terminaison de la ligne de coupe (22) suivant une ligne s'étendant obliquement vers l'extérieur sur une distance (17) correspondant sensiblement à la longueur de la partie de jambe supérieure du patient et en ce que le reste du bord supérieur (15) de chaque jambière, qui est sensiblement parallèle au bord inférieur du tissu, est rattachée le long des sections de bord supérieures (15) jusqu'aux extrémités fermées des jambières.

4. Tissu selon la revendication 3, caractérisé en ce que les ouvertures opératoires (19, 23) sont ménagées entre les jambières ainsi que dans la partie rectangulaire (18) située dans le voisinage des jambières ; et en ce que le rabat (20, 21) est destiné à être appliqué par-dessus l'une ou l'autre des deux ouvertures opératoires selon qu'il y a à pratiquer une incision chirurgicale dans l'urètre/vagin ou dans l'abdomen.

5. Procédé de fabrication de tissu chirurgical selon la revendication 1 devant servir pendant incision chirurgicale de l'urètre/vagin ou de l'abdomen, caractérisé en ce qu'on forme le champ d'une seule ébauche oblongue pliée autour de sa médiane transversale, l'extrémité de pliage continue obtenue étant pliée vers l'intérieur pour former deux jambières (13, 14) dimensionnées de façon que chacune des jambes du patient puisse être reçue dans chaque jambière, l'ébauche étant ensuite cisaillée ou sectionnée longitudinalement dans le sens s'étendant à partir de son extrémité (12) demeurée ouverte du fait du pliage parallèlement à la direction longitudinale de l'ébauche et allant au moins sensiblement jusqu'aux jambières (13, 14) ; en ce qu'on réunit l'ébauche pliée suivant le bord cisaillé ou coupé (16) ; et en ce qu'on joint individuellement les jambières (13, 14) audit bord coupé (16) obliquement vers l'extérieur à partir d'un bord de l'ébauche ou jambière et en outre suivant les bords rectilignes (15) jusqu'aux extrémités fermées des jambières, au moins une ouverture opératoire traversante (19) étant ménagée dans l'ébauche.

6. Procédé selon la revendication 5, caractérisé en ce que le tissu est formé d'une ébauche rectangulaire ; en ce que les parties (20, 21) situées au-dessus de la ligne de coupe (16) sont réunies (22) suivant cette forme pour constituer un rabat continu (20, 21) destiné à recouvrir soit une ouverture opératoire (19) située à l'entrejambe des jambières, soit une ouverture opératoire (3) ménagée dans la partie rectangulaire (18).

7. Procédé selon la revendication 5, caractérisé en ce que l'ébauche, après avoir été cisaillée ou sectionnée longitudinalement dans le sens s'étendant depuis l'extrémité de l'ébauche (12) demeurée ouverte du fait du pliage, parallèlement à la direction longitudinale de l'ébauche, et allant au moins sensiblement jusqu'aux jambières (13, 14), est ensuite cisaillée ou sectionnée obliquement vers un bord (15) de l'ébauche et de chaque jambière, l'ébauche pliée étant ensuite rattachée suivant le bord cisaillé ou coupé (16) ; et en ce que les jambières (13, 14) sont individuellement réunies suivant les bords obliques (17) et en outre suivant les bords rectilignes (15) jusqu'aux extrémités fermées des jambières, avec formation dans l'ébauche d'une ouverture opératoire traversante (19) qui dans le tissu chirurgical achevé est située entre les jambières, principalement à l'entrejambe des jambières.

FIG.1

3

7    5    2

8              6    4

1

FIG.2

8

5              7              1

6

3

3'              4

2         4'

10    9    11

FIG.3

FIG. 4

## FIG.5

15

17

22

20

13

18

12

## FIG.6

15

14

17

23

21

22

19

20

17

13

15

18

FIG. 7

FIG. 8